(19) Europäisches Patentamt
European Patent Office
Office européen des brevets

(11) Publication number: **0 052 425 A2**

(12) **EUROPEAN PATENT APPLICATION**

(21) Application number: 81304836.0

(22) Date of filing: 16.10.81

(51) Int. Cl.[3]: **C 07 D 233/60**
C 07 D 249/08, C 07 D 303/04
C 07 D 401/06, C 07 D 405/06
C 07 D 409/06, A 01 N 43/50
A 01 N 43/64

(30) Priority: 13.11.80 GB 8036438

(43) Date of publication of application:
26.05.82 Bulletin 82/21

(84) Designated Contracting States:
AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Worthington, Paul Anthony
4 Oakhurst Road Maidenhead Court Park
Maidenhead Berkshire, SL6 8HY(GB)

(72) Inventor: de Fraine, Paul
77 McCarthy Way
Wokingham Berkshire(GB)

(74) Representative: Alner, Henry Giveen Hamilton et al,
Imperial Chemical Industries PLC Legal Department: Patents Thames House North Millbank
London SWIP 4QG(GB)

(54) **Triazole and imidazole compounds, a process for preparing them, their use as plant fungicides and fungicidal compositions containing them.**

(57) Compounds of the formula:

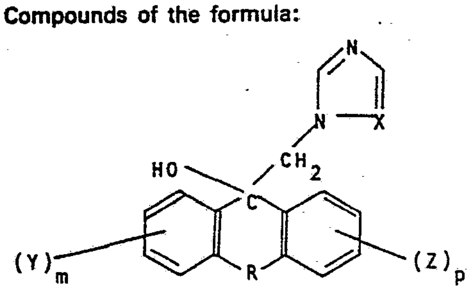

having pesticidal (especially fungicidal) and plant growth regulating activity wherein R is a bridging group $-(CH_2)_n-$ where n is 0, 1 or 2, or a bridging group $-CH=CH-$, or is $-O-$, $-S-$, $-NH-$, or

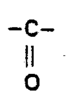 ;

X is =N− or =CH−; Y and Z, which may be the same or different, are halogen, alkyl, alkoxy, halo-alkoxy, halo-alkyl, nitro, phenyl or phenoxy; m and p are 0, 1, 2 or 3; and salts, metal complexes and functional derivatives thereof.

EP 0 052 425 A2

Croydon Printing Company Ltd.

# TRIAZOLE AND IMIDAZOLE COMPOUNDS, A PROCESS FOR PREPARING THEM, THEIR USE AS PLANT FUNGICIDES AND FUNGICIDAL COMPOSITIONS CONTAINING THEM

This invention relates to triazole and imidazole compounds useful as fungicides, to a process for preparing them, to fungicidal compositions containing them, and to a method of combating fungal infections in plants using them.

The triazole and imidazole compounds have the general formula (I):

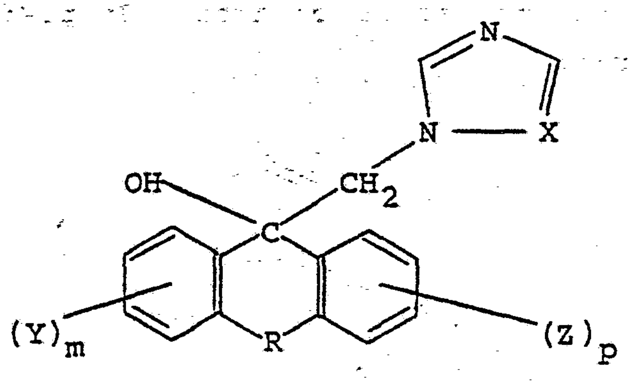

(I)

wherein R is a bridging group $-(CH_2)_n-$ where n is 0, 1 or 2, or a bridging group -CH=CH-, or is -O-, -S-, -NH- or $-\underset{\underset{O}{\|}}{C}-$; X is =N- or =CH-; Y and Z, which may be the same or different, are halogen, alkyl, alkoxy, halo-alkoxy, halo-alkyl, nitro, phenyl or phenoxy; m and p are 0, 1, 2 or 3; and salts, metal complexes and functional derivatives thereof.

The compounds of the invention can contain optical isomers. Such compounds are generally obtained in the form of racemic mixtures. However, these mixtures can be separated into the individual isomers by methods known in the art.

Examples of suitable substituents for the phenyl rings are halogen (e.g. fluorine, chlorine or bromine), $C_{1-5}$ alkyl [e.g. methyl, ethyl, propyl (n- or *iso*-propyl) and

butyl (n-, sec-, iso- or t-butyl)], $C_{1-4}$ alkoxy (e.g. methoxy and ethoxy), halo-alkoxy (e.g. trifluoromethoxy), trifluoromethyl, nitro, phenyl and phenoxy. Suitably the phenyl is unsubstituted or substituted with one, two or three ring substituents as defined above.

The salts can be salts with inorganic or organic acids e.g. hydrochloric, nitric, sulphuric, acetic, p-toluenesulphonic or oxalic acid.

Suitably the metal complex is one including, as the metal, copper, zinc, manganese or iron. It preferably has the general formula:

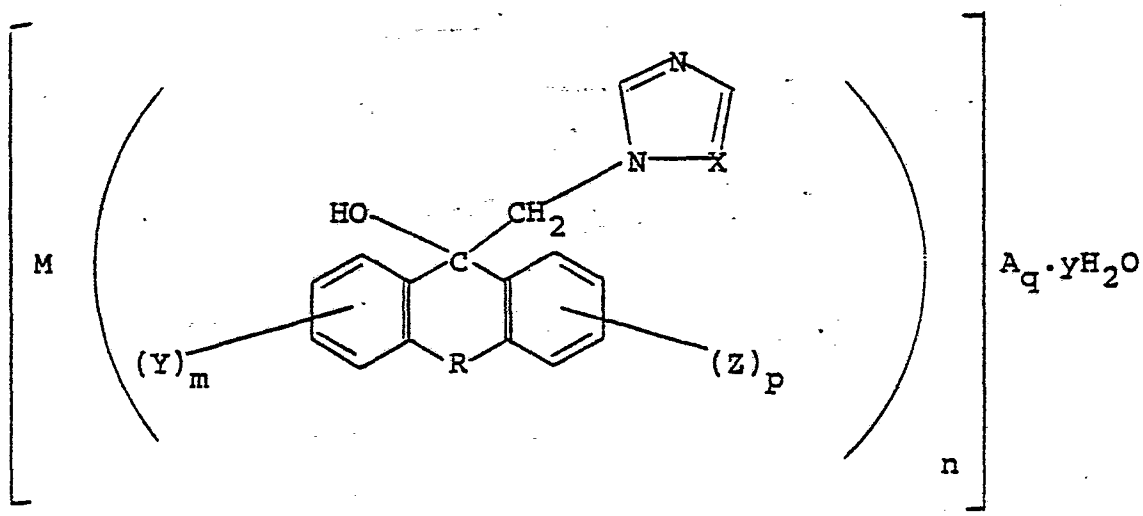

wherein R, X, Y, Z, m and p are as defined above, M is a metal, A is an anion (e.g. a chloride, bromide, iodide, nitrate sulphate or phosphate anion), n is 2 or 4 and y is 0 or an integer of 1 to 12, and q is an integer consistent with valency.

Examples of the compounds of the invention are shown in Table I and conform to the general formula I when m and p are both 0 in each case.

| COMPOUND NO | R | X | MELTING POINT (°C) |
| --- | --- | --- | --- |
| 1 | —— | =N- | 105-6 |
| 2 | -CH=CH- | =N- | 164-5 |
| 3 | -CH=CH- | =CH- | 234-5 |
| 4 | $-CH_2-CH_2-$ | =N- | 155-6 |
| 5 | $-CH_2-CH_2-$ | =CH- | 168-9 |
| 6 | —— | =CH- | 193-5 |

Note "——" for compound Nos 1 and 6 signifies a straight bond connecting the two carbon atoms of the phenyl groups. In other words it is $-(CH_2)_n-$ with n=O.

The invention is also illustrated by the compound:-

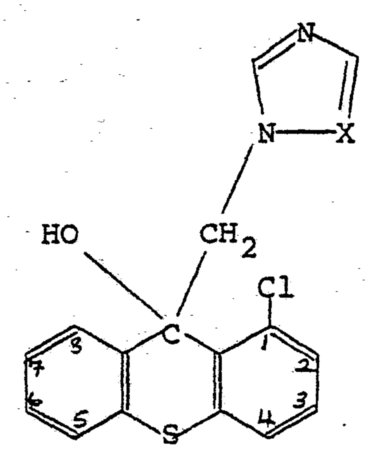

and the numbering of the ring atoms is as shown

The compounds of general formula (I) may be produced by reacting a compound of general formula (II)

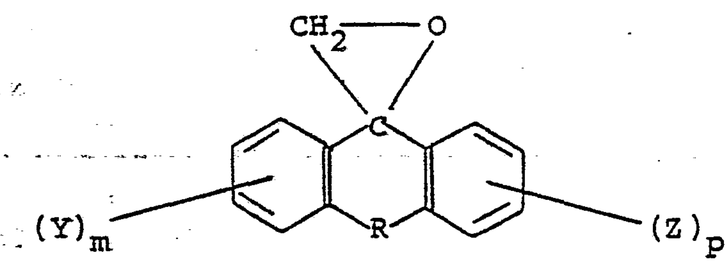

(II)

in which R, Y, Z, m and p are as defined above with 1,2,4-triazole or imidazole either in the presence of an acid-binding agent or in the form of one of its alkali metal salts in a convenient solvent.

The above compounds of formula II are novel and useful intermediates and are deemed, per se, to form part of the present invention.

Suitably the compound of general formula (II) is reacted at 20-100°C with the sodium salt of 1,2,4-triazole or imidazole (the salt can be prepared by adding either sodium hydride or sodium methoxide to 1,2,4-triazole) in a convenient solvent such as acetonitrile, methanol, ethanol or dimethylformamide. The product can be isolated by pouring the reaction mixture into water and recrystallising the solid formed from a convenient solvent.

The compounds of general formula (II) wherein R, Y, Z, m and p are as defined above may be prepared by reacting the appropriate ketone compound of general formula (III)

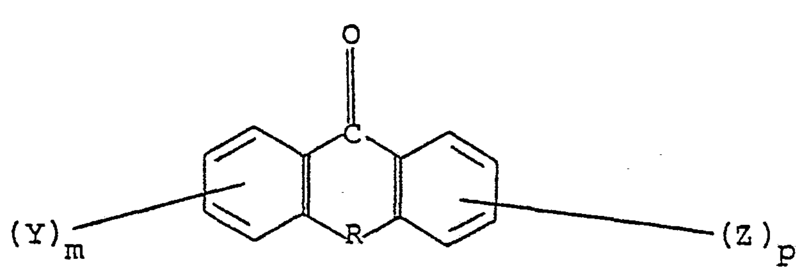

(III)

wherein R, Y, Z, m and p are as defined above, with dimethyl oxosulphonium methylide (Corey and Chaykovsky, JACS, 1965, 87, 1353-1364) or dimethyl sulphonium methylide (Corey and Chaykovsky, JACS, 1962, 84, 3782) using methods set out in the Literature i.e. J.M. Muchowski et al Can J Chem 1969, 47, 4327-33 and M.A. Davis et al J Med Chem 1967, 10, 627-35.

The salts and metal complexes of the compounds of general formula (I) can be prepared from the latter in known manner. For example, the complexes can be made by reacting the uncomplexed compound with a metal salt in a suitable solvent.

The compounds, salts and metal complexes are active fungicides, particularly against the diseases:-

Piricularia oryzae on rice
Puccinia recondita, Puccinia striiformis and other rusts on wheat, Puccinia hordei, Puccinia striiformis and other rusts on barley, and rusts on other hosts e.g. coffee, apples, vegetables and ornamental plants
Plasmopara viticola on vines
Erysiphe graminis (powdery mildew) on barley and wheat and other powdery mildews on various hosts such as Sphaerotheca fuliginea on cucurbits (e.g. cucumber), Podosphaera leucotricha on apples and Uncinula necator on vines
Helminthosporium spp. and Rhynchosporium spp. on cereals
Cercospora arachidicola on peanuts and other Cercospora species on for example sugar beet, bananas and soya beans
Botrytis cinerea (grey mould) on tomatoes, strawberries, vines and other hosts
Phytophthora infestans (late blight) on tomatoes
Venturia inaequalis (scab) on apples

Some of the compounds have also shown a broad range of activities against fungi in vitro. They have activity against various post-harvest diseases on fruit (e.g. Penicillium digatatum and italicum on oranges and

Gloeosporium musarum on bananas). Further some of the compounds are active as seed dressings against: Fusarium spp., Septoria spp., Tilletia spp. (i.e. bunt, a seed borne disease of wheat), Ustilago spp., Helminthosporium spp. on cereals, Rhizoctonia solani on cotton and Corticium sasakii on rice.

The compounds can move acropetally in the plant tissue. Moreover, the compounds can be volatile enough to be active in the vapour phase against fungi on the plant.

The compounds are also useful for the treatment of candidiasis and human dermatophyte infections.

The compounds also possess plant growth regulating properties and are useful for this further purpose.

The compounds can also have plant growth regulating activities.

The plant growth regulating effects of the compounds are manifested as for example a stunting or dwarfing effect on the vegetative growth of woody and herbaceous mono- and di-cotyledonous plants. Such stunting or dwarfing may be useful, for example, in peanuts, cereals and soya bean where reduction in stem growth may reduce the risk of lodging and may also permit increased amounts of fertiliser to be applied. The stunting of woody species is useful in controlling the growth of undergrowth under power lines etc. Compounds which induce stunting or dwarfing may also be useful in modifying the stem growth of sugar cane thereby increasing the concentration of sugar in the cane at harvest; in sugar cane, the flowering and ripening may be controllable by applying the compounds. Stunting of peanuts can assist in harvesting. Growth retardation of grasses can help maintenance of grass swards. Examples of suitable grasses are Stenotaphrum secundatum (St. Augustine grass), Cynosurus cristatus, Lolium multiflorum and perenne, Agrostis tenuis, Cynodon dactylon (Bermuda grass), Dactylis glomerata, Festuca spp. (e.g. Festuca rubra) and Poa spp. (e.g. Poa pratense). The compounds may stunt grasses

and can therefore be used on grass verges. They may also have an effect on flower head emergence in for example grasses. The growth of non-crop vegetation (e.g. weeds or cover vegetation) can be retarded thus assisting in the maintenance of plantation and field crops. In fruit orchards, particularly orchards subject to soil erosion, the presence of grass cover is important. However excessive grass growth requires substantial maintenance. The compounds of the invention could be useful in this situation as they could restrict growth without killing the plants which would lead to soil erosion; at the same time the degree of competition for nutrients and water by the grass would be reduced and this could result in an increased yield of fruit. In some cases, one grass species may be stunted more than another grass species; this selectivity could be useful for example for improving the quality of a sward by preferential suppression of the growth of undesirable species.

As indicated above, the compounds can also be used to stunt woody species. This property can be used to control hedgerows or to shape fruit trees (e.g. apples). Some coniferous trees are not significantly stunted by the compounds so the compounds could be useful in controlling undesirable vegetation in conifer nurseries.

In the potato, vine control in the field and inhibition of sprouting in the store may be possible.

Other plant growth regulating effects caused by the compounds include alteration of leaf angle and promotion of tillering in monocotyledonous plants. The former effect may be useful for example in altering the leaf orientation of, for example, potato crops thereby letting more light into the crops and inducing an increase in phytosynthesis and tuber weight. By increasing tillering in monocotyledonous crops (e.g. rice), the number of flowering shoots per unit area may be increased thereby increasing the overall grain yield of such crops. In

grass swards an increase in tillering could lead to a denser sward which may result in increased resilience in wear.

The treatment of plants with the compounds can lead to the leaves developing a darker green colour.

The compounds may inhibit, or at least delay, the flowering of sugar beet and thereby may increase sugar yield. They may also reduce the size of sugar beet without reducing significantly the sugar yield thereby enabling an increase in planting density to be made. Similarly in other root crops (e.g. turnip, swede, mangold, parsnip, beetroot, yam and cassava) it may be possible to increase the planting density.

The compounds could be useful in restricting the vegetative growth of cotton thereby leading to an increase in cotton yield.

The compounds may be useful in rendering plants resistant to stress since the compounds can delay the emergence of plants grown from seed, shorten stem height and delay flowering; these properties could be useful in preventing frost damage in countries where there is significant snow cover in the winter since then the treated plants would remain below snow cover during the cold weather. Further the compounds may cause drought or cold resistance in certain plants.

When applied as seed treatments at low rates the compounds can have a growth stimulating effect on plants.

In carrying out the plant growth regulating method of the invention, the amount of compound to be applied to regulate the growth of plants will depend upon a number of factors, for example the particular compound selected for use, and the identity of the plant species whose growth is to be regulated. However, in general an application rate of 0.1 to 15, preferably 0.1 to 5, kg per hectare is used. However, on certain plants even application rates within these ranges may give undesired

phytotoxic effects. Routine tests may be necessary to determine the best rate of application of a specific compound for any specific purpose for which it is suitable.

The compounds may be used as such for fungicidal purposes but are more conveniently formulated into compositions for such usage. The invention thus provides also a fungicidal or plant growth regulating composition comprising a compound of general formula (I), or a salt or metal complex or functional derivative thereof as hereinbefore defined, and a carrier or diluent therefor.

The invention also provides a method of combating fungal diseases in a plant, which method comprises applying to the plant, to seed of the plant, or to the locus of the plant or seed, a compound, or a salt or metal complex or functional derivative thereof as hereinbefore defined, or a composition containing the same.

The compounds also possess, as stated above, plant growth regulating activity. The invention therefore further provides a method of regulating the growth of a plant which method comprises applying to the plant, to the seed of the plant, or to the locus of the plant or seed, a compound, or a salt or metal complex, or functional derivative thereof, or a composition containing the same.

The compounds, salts and complexes can be applied in a number of ways, for example they can be formulated or unformulated, directly to the foliage of a plant, to seeds or to other medium in which plants are growing or are to be planted, or they can be sprayed on, dusted on or applied as a cream or paste formulation, or they can be applied as a vapour. Application can be to any part of the plant, bush or tree, for example to the foliage, stems, branches or roots, or to soil surrounding the roots, or to the seed before it is planted.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene

dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (e.g. 2-ethoxyethanol and 2-butoxyethanol).

The compositions to be used as sprays may also be in the form of aerosols wherein the formulation is held in a container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form.

By including suitable additives, for example additives for improving the distribution, adhesive power and resistance to rain on treated surfaces, the different compositions can be better adapted for various utilities.

The compounds can be used as mixtures with fertilisers (e.g. nitrogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the compound of general formula (I) or a salt or metal complex thereof.

The compositions may also be in the form of liquid preparations for use as dips or sprays which are generally aqueous dispersions or emulsions containing the active ingredient in the presence of one or more surfactants e.g. wetting agent(s), dispersing agent(s), emulsifying agent(s) or suspending agent(s). These agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropyl-naphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), the concentrate to be diluted with water before use. These concentrates often should be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 10-85%, for example 25-60%, by weight of the active ingredient(s). These concentrates suitably contain organic acids (e.g. alkaryl or aryl sulphonic acids such as xylenesulphonic acid or dodecyl benzenesulphonic acid) since the presence of such acids can increase the solubility of the active ingredient(s) in the polar solvents often used in the concentrates. The concentrates suitably contain also a high proportion of surfactants so that sufficiently stable

emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, e.g. compounds having similar or complementary fungicidal activity or compounds having plant growth regulating, herbicidal or insecticidal activity.

The other fungicidal compound can be for example one which is capable of combating ear diseases of cereals (e.g. wheat) such as *Septoria*, *Gibberella* and *Helminthosporium* spp., seed and soil borne diseases and downy and powdery mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compound are imazalil, benomyl, carbendazim, thiophanate-methyl, captafol, captan, sulphur, triforine, dodemorph, tridemorph, pyrazophos, furalaxyl, ethirimol, dimethirimol, bupirimate, chlorothalonil, vinclozolin, procymidone, iprodione, metalaxyl, forsetyl-aluminium, carboxin, oxycarboxin, fenarimol, nuarimol, fenfuram, methfuroxan, nitrotal-isopropyl, triadimefon, thiabendazole, etridiazole, triadimenol, biloxazol, dithianon, binapacryl, quinomethionate, guazitine, dodine, fentin acetate, fentin hydroxide, dinocap, folpet, dichlofluanid, ditalimphos, kitazin, cycloheximide, dichlobutrazol, a dithiocarbamate, a copper compound, a mercury compound, 1-(2-cyano-2-methoxyiminoacetyl)-3-ethyl urea, fenapanil, ofurace, propioconazole, etaconazole and fenpropemorph.

The compounds of general formula (I) can be mixed with soil, peat or other rooting media for the protection of plants against seed-borne, soil-borne or foliar fungal diseases.

Suitable insecticides are Pirimor, Croneton, dimethoate, Metasystox and formothion.

Examples of suitable plant growth regulating compounds are the gibberellins (e.g. $GA_3$, $GA_4$ or $GA_7$, the auxins (e.g. indoleacetic acid, indole-butyric acid, naphthoxyacetic acid or naphthylacetic acid), the cytokinins (e.g. kinetin, diphenylurea, benzimidazole, benzyladenine or benzylaminopurine), phenoxyacetic acids (e.g. 2,4-D or MCPA), substituted benzoic acids (e.g. triiodobenzoic acid), morphactins (e.g. chlorfluorecol), maleic hydrazide, glyphosate, glyphosine, long chain fatty alcohols and acids, dikegulac, fluoridamid, mefluidide, substituted quaternary ammonium and phosphonium compounds (e.g. chlormequat or chlorphonium), ethepon, carbetamide, methyl-3,6-dichloranisate, daminozide, asulam, abscissic acid, isopyrimol, 1(4-chlorophenyl)-4,6-dimethyl-2-oxo-1,2-dihydropyridine-3-carboxylic acid, hydroxybenzonitriles (e.g. bromoxynil), difenzoquat, benzoylpropethyl-3,6-dichloropicolinic acid.

The following Example illustrates the invention; the temperatures are given in degrees Centigrade (°C).

EXAMPLE 1

This Example illustrates the preparation of compound No.2 of Table I above.

A suspension of sodium hydride (0.1 mol) in dry dimethylsulphoxide (D.M.S.O; 100 ml) was heated to 50° for 3 hours under nitrogen. Dry tetrahydrofuran (THF; 100 ml) was added and the solution was cooled to -10°. Trimethylsulphonium iodide (0.1 mole) dissolved in DMSO (80 ml) was added dropwise at -10°. After stirring for 1 minute at -5° a solution of 5H-dibenzo[a,d]-cyclohepten-5-one (0.09 mol) in DMSO (20 ml) and THF (20 ml) was added dropwise at -5°. The solution was stirred for 30 minutes at 0° and 1 hour at room temperature, then poured into water. The solution was extracted with diethyl ether (400 ml) washed with water (4 x 300 ml) and dried over anhydrous magnesium sulphate. Removal of the solvent gave an oil which was triturated with petroleum ether (60-80°) and filtered to give the spiro (oxirane-2,5'-dibenzo-5H-suberene) (90%) as a white crystalline solid m.p. 89°C.

1,2,4-Triazole (0.04 mol) was added portionwise to sodium hydride (0.04 mol) in DMF (40 ml) and the solution stirred at room temperature until effervescence ceased. The oxirane (0.02 mol) in DMF (10 ml) was added dropwise and the solution stirred at 140° for 2 hours. The solution was cooled to room temperature and poured into water to give a white solid which was filtered off washed with water and dried. Recrystallisation from petroleum (60-80°) chloroform, methanol, gave the title compound (70%) as a white crystalline solid m.p. 164-5°C.

EXAMPLE 2

This Example illustrates the preparation of Compound No.3 of Table I above.

Sodium hydride (0.04 mol - 50% suspension in oil) was suspended in DMF (40 ml) and imidazole (0.04 mol) was added portionwise. The solution was stirred at room temperature until all the effervesence had ceased. The epoxide (0.02 mol) prepared in Example 1 was dissolved in DMF (10 ml) and added dropwise to the above solution. This solution was warmed at 120° for 2 hours, poured into water (200 ml) to give a crystalline solid which recrystallised from petroleum ether/chloroform/methanol solution to give the title compound (85%) m.p. 234-5°C.

EXAMPLE 3

The compounds were tested against a variety of foliar fungal diseases of plants. The technique employed was as follows.

The plants were grown in John Innes Potting Compost (No 1 or 2) in 4 cm diameter minipots. A layer of fine sand was placed at the bottom of the pots containing the dicotyledonous plants to facilitate uptake of test compound by the roots. The test compounds were formulated either by bead milling with aqueous Dispersol T or as a solution in acetone or acetone/ethanol which was diluted to the required concentration immediately before use. For the foliage diseases, suspensions (100 ppm active ingredient) were sprayed on to the soil. Exceptions to this were the tests on Botrytis cinerea, Plasmopara viticola and Venturia inaequalis. The sprays were applied to maximum retention and the root drenches to a final concentration equivalent to approximately 40 ppm a.i./dry soil. Tween 20, to give a final concentration of 0.05%, was added when the sprays were applied to cereals.

For most of the tests the compound was applied to the soil (roots) and to the foliage (by spraying) one or two days before the plant was inoculated with the diseases. An exception was the test on <u>Erysiphe graminis</u> in which the plants were inoculated 24 hours before treatment. After inoculation, the plants were put into an appropriate environment to allow infection to take place and then incubated until the disease was ready for assessment. The period between inoculation and assessment varied from four to fourteen days according to the disease and environment.

The disease control was recorded by the following grading:-

4 = no disease
3 = trace - 5% of disease on untreated plants
2 = 6-25% of disease on untreated plants
1 = 26-59% of disease on untreated plants
0 = 60-100% of disease on untreated plants

The results are shown in Table II.

TABLE II

| COMPOUND NUMBER | PUCCINIA RECONDITA (WHEAT) | ERYSIPHE GRAMINIS (BARLEY) | PIRICULARIA ORYZAE (RICE) | PLASMOPARA VITICOLA (VINE) | PHYTOPHTHORA INFESTANS (TOMATO) | BOTRYTIS CINEREA (TOMATO OR GRAPE) | CERCOSPORA ARACHIDICOLA (PEANUT) | VENTURIA INAEQUALIS (APPLE) |
|---|---|---|---|---|---|---|---|---|
| 1 | 3 | 4 | – | 0 | 1 | 0 (tomato) | 0 | 1 |
| 2 | 4 | 4 | – | 0 | 0 | 4 (grape) | 4 | 3 |
| 3 | 2 | 4 | – | 0 | 0 | 0 (grape) | 1 | 2 |
| 4 | 4 | 4 | – | 0 | 0 | 0 (grape) | 4 | 4 |
| 5 | | 4 | – | 0 | 0 | 1 (grape) | 3 | 1 |
| 6 | 0 | 4 | – | 0 | 0 | 2 (grape) | 0 | 0 |

"–" means not tested.

EXAMPLE 4

This Example illustrates the plant growth regulating properites of the compounds on a range of monocotyledonous and dicotyledonous plants. The compounds were applied as an overall spray to the foliage of young seedlings grown in peat compost. After 12 days the plants were assessed for plant growth regulating effects and phytotoxic symptoms. Table III shows the stunting effect of the compounds on the vegetative growth using the following grading:-

1 = 0 - 30% retardation
2 = 31 - 75% retardation
3 = 75% retardation

Additional plant growth regulating properties are indicated as follows:-

G = darker green leaf colour
A = apical effect
T = tillering/sideshooting effect

0052425

TABLE III

| COMPOUND NUMBER | DAYS AFTER TREATMENT | RATE PPM | AT | CC | DA | BR | WW | MZ | CT | LT | SB | SY | TO | RA |
|---|---|---|---|---|---|---|---|---|---|---|---|---|---|---|
| 1 | 12 | 2000 | 1 | 1 | 1 | 0 | 0 | 2 | 2A | 2 | 2 | 1A | 1 | 1 |
| 2 | 12 | 2000 | 3 | 3 | 3 | 1GT | 1 | 3 | 3A | 3A | 3GA | 3AT | 3A | 3A |
| | | 600 | 3 | 3 | 2 | 1 | 1 | 3 | 3A | 3A | 3G | 3AT | 3A | 3A |
| | | 200 | 1 | 1 | 1 | 0 | 0 | 3 | 3A | 3A | 3G | 3A | 3A | 3A |
| 3 | 12 | 2000 | 3 | 3 | 3 | 0 | 0 | 0 | 2 | 2 | 2A | 2A | 3A | 2A |
| 4 | 12 | 2000 | 1 | 1 | 1 | 0 | 0 | 2 | 3A | 2 | 3A | 1 | 0 | 0 |
| 6 | 12 | 2000 | 1 | 1 | 1 | 0 | 0 | 0 | 1 | 2 | 2 | 0 | 1 | 1 |

Plant species:
AT = Agrostis tenuis
CC = Cynosurus cristatus
DA = Dactylis glomerata
BR = Barley (cv. Sundance)
WW = Wheat (cv. Sappo)
MZ = Maize (cv. Proneer 3369)
CT = Cotton (cv. Delta Pine Gl)
LT = Lettuce (cv. Chobham Green)
SB = Sugar beet (cv. Amono)
SY = Soya (cv. Amsoy)
TO = Tomato (Ailsa Craig)
RA = Radish (cv. Star)

EXAMPLE 5

This Example illustrates the retardation properties of Compound 1 on a range of grass species. The compound was formulated as an emulsifiable concentrate and sprayed on 4" pots of grass trimmed to approximately 1" 27 days after sowing. The regrowth was assessed 22 days after spraying. Retardation was assessed on a 0-3 scale as described for example 4. Phytotoxicity was scored on a similar 0-3 scale. The results are shown in Table IV.

TABLE IV

The effect of compound 1 on various grass species

| RATE kg $ha^{-1}$ | ASSESSMENT | SPECIES | | | | | | TOTAL |
|---|---|---|---|---|---|---|---|---|
| | | AT | FR | CD | LP | DG | PP | |
| 2.0 | Retardation | 1 | 0 | 3 | 3 | 2 | 1 | 10 |
| | Phytotoxicity | 2 | 0 | 0 | 1 | 1 | 0 | 4 |
| 0.6 | Retardation | 0 | 0 | 0 | 2 | 0 | 0 | 2 |
| | Phytotoxicity | 0 | 0 | 0 | 0 | 0 | 0 | 0 |

Species Code: AT = Agrostis tenuis = Common bent
FR = Festuca rubra = Red Fescue
CD = Cynodondactylon = Bermuda grass
LP = Lolium perenne = Perennial ryegrass
DG = Dactylisglomerata = Cocksfoot
PP = Poa pratensis = Smooth meadow grass

1. Triazole and imidazole compounds having the general formula (I):

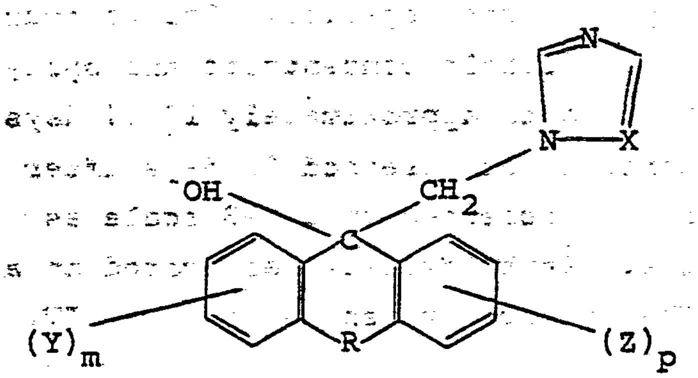

(I)

wherein R is a bridging group $-(CH_2)_n$ where n is 0, 1 or 2, or a bridging group $-CH=CH-$, or is $-O-$, $-S-$, $-NH-$ or $-\underset{\underset{O}{\|}}{C}-$; X is $=N-$ or $=CH-$; Y and Z, which may be the same or different, are halogen, alkyl, alkoxy, halo-alkoxy, halo-alkyl, nitro, phenyl or phenoxy, m and p are 0, 1, 2 or 3; and salts, metal complexes and functional derivatives thereof.

2. Compounds as claimed in claim 1 wherein R is $-CH=CH-$ or $-CH_2-CH_2-$ or is a straight bond between the two ring carbon atoms; and m and p are 0.

3. The specific compounds set out in Table I of the foregoing description.

4. A process for making the compounds of general formula (I) as defined in claims 1 and 2 which comprises reacting a compound of general formula (II)

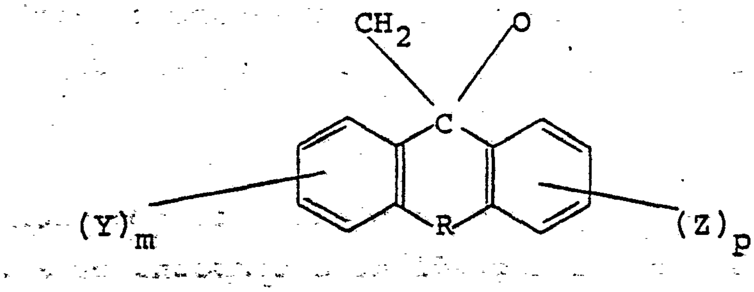

(II)

in which R, Y, Z, m and p are as defined above with 1,2,4-triazole or imidazole either in the presence of an acid-binding agent or in the form of one of its alkali metal salts in a convenient solvent.

5. A process according to claim 4 wherein the compound of general formula (II) is reacted at 20-100°C with the sodium salt of 1,2,4-triazole or imidazole (the salt can be prepared by adding either sodium hydride or sodium methoxide to 1,2,4-triazole) in a convenient solvent such as acetonitrile, methanol, ethanol or dimethylformamide, and thereafter, if necessary, isolating the product by pouring the reaction mixture into water and recrystallising the solid formed from a convenient solvent.

6. A process according to claim 4 or 5 wherein the compounds of general formula (II) wherein R, Y, Z, m and p are as defined above are prepared by reacting the appropriate ketone compound of general formula (III)

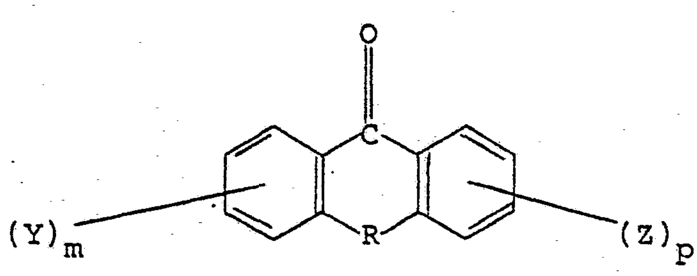

(III)

wherein R, Y, Z, m and p are as defined above, with dimethyl oxosulphonium methylide or dimethyl sulphonium methylide.

7. A fungicidal or plant growth regulating composition comprising as an active ingredient a compound or a salt or complex thereof, as defined in any of claims 1 to 3 and a carrier or diluent therefor.

8. A method of combating fungal diseases in a plant, which method comprises applying to the plant, to seed of the plant, or to the locus of the plant or seed, a compound, or a salt or metal complex or functional derivative thereof, as defined in any of claims 1 to 3 or a composition as defined in claim 7.

9. A method of regulating the growth of a plant which method comprises applying to the plant, to the seed of the plant, or to the locus of the plant or seed, a compound, or a salt or metal complex or functional derivative thereof, as defined in any of claims 1 to 3 or a composition as defined in claim 7.

10. As novel intermediates the compounds having the general formula:

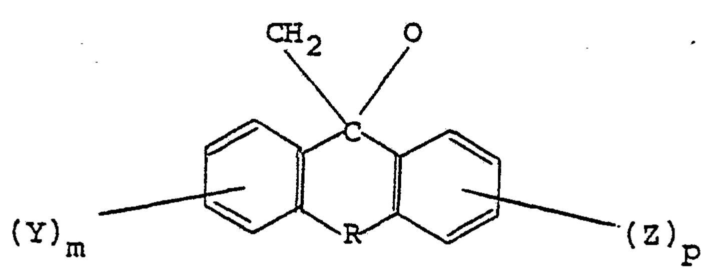

wherein R, Y, Z, m and p are as defined in claim 1.